# EUROPEAN PATENT SPECIFICATION

(11) **EP 0 283 505 B1**
(45) Date of publication and mention of the grant of the patent: **06.07.1994**
(21) Application number: 87906496.2
(22) Date of filing: 19.08.1987
(51) Int. Cl.: A61K 39/02, A61K 39/12, A61K 39/295, A61K 39/385

(54) **VACCINE AND METHOD OF PREPARATION**
IMPFSTOFF UND VERFAHREN ZUR HERSTELLUNG
VACCIN ET PROCEDE DE PREPARATION

(30) Priority: 22.09.1986 US 909964; 16.07.1987 US 75187
(43) Date of publication of application: 28.09.1988
(73) Proprietor: EMORY UNIVERSITY, Atlanta, GA 30322 (US)
(72) Inventor: HUNTER, Robert, L., Tucker, GA 30084 (US)
(74) Representative: Sternagel, Hans-Günther, Dr.
(86) International application number: US8702056
(87) International publication number: WO8801873

(56) References cited:
- US-A- 2 674 619
- US-A- 2 979 528
- US-A- 3 022 335
- US-A- 3 036 118
- US-A- 4 372 945
- US-A- 4 400 376
- US-A- 4 478 823
- US-A- 4 503 036
- US-A- 4 711 876
- CHEMICAL ABSTRACTS, vol. 105, no. 1, July 7, 1986, Columbus, OH (US); N.Y. ALEKSEEVA et al., p. 450, no. 4730x#
- JOURNAL OF EXPERIMENTAL MEDICINE, vol. 134, 1971; FELDMAN, pp. 103-119#
- NEW ENGLAND JOURNAL OF MEDICINE, vol. 315, no. 10, 04 September 1986; HOFFMAN, pp. 601-606#
- ARCH. OF BIOCHEMISTRY AND BIOPHYSICS, vol. 84, 1959; KOBAYASHI, pp. 342-362#

## Description

### Technical Field

The present invention relates to a vaccine and more particularly to a vaccine comprising a bacterial flagella which, when conjugated with an antigen moiety, amplifies the immune response to the antigen.

### Background Art

A vaccine is defined herein as a suspension of antigenic moieties, usually consisting of infectious agents, or some part of the infectious agents, that is injected into the body to produce active immunity. The antigenic moiety making up the vaccine can be either a natural product purified from a microorganism, a synthetic product or a genetically engineered protein peptide or similar product. An adjuvant is defined herein as any substance whose admixture with an injected immunogen increases the immune response. A hapten is defined herein as a substance that reacts selectively with appropriate antibodies but the hapten itself is usually not immunogenic. Most haptens are small molecules, but some macromolecules can also function as haptens. Conjugation is defined herein as the covalent or other form of linking of two or more molecules.

Sixty years ago it was demonstrated that it was possible to augment the antitoxin response to diphtheria and tetanus by administering vaccines as a mixture with pyogenic bacteria or with various additional compounds. Since that time, clinicians and immunologists have sought to potentiate the immune response with adjuvants while attempting to minimize the often-present side effects.

Biosynthetic and recombinant DNA technology is permitting development of vaccines possessing antigenic epitopes that were previously impossible to produce. Current vaccine candidates, by way of example, include synthetic peptides that immunogenically mimic streptococcal, gonococcal, hoof and mouth disease, AIDS (HIV-1 virus) and malarial antigens.

The work on the parasitic disease malaria is especially important. This disease affects in excess of 200,000,000 people per year worldwide and is the most important disease in the world in terms of morbidity and loss of work. The techniques of genetic engineering have been used to identify, and now to produce in substantial quantities, several proteins of malarial parasites. In particular, a twelve amino acid peptide from the sporozoite stage has been determined to carry an important antigenic site. Antibodies against this particular peptide can kill the parasite immediately after it is injected. Unfortunately, this peptide, by itself, does not produce an adequate immune response.

In an effort to induce an effective immune response to the sporozoite peptide, the peptide has been administered with adjuvants. To date, however, the adjuvants used with the peptide have not produced satisfactory results. Thus, interest has arisen in the development of potent, nontoxic adjuvants that will enhance the immunogenicity of haptenic epitopes. In addition, adjuvants are needed for use with conventional vaccines to elicit an earlier, more potent, or more prolonged response. Such an adjuvant would also be useful in cases where antigen supply is limited or is costly to produce.

The development of adjuvants has, until recently, been empirical. An enormous number of compounds have been found to modulate the immune response. These compounds have been notably diverse in both substance and function, a fact that has complicated attempts to discover the unifying mechanisms of adjuvant action. The elucidation of these mechanisms has lagged behind recent advances in the understanding of the immune system.

This diversity of adjuvants has presented difficulties in their classification. Adjuvants are occasionally grouped according to their origin, be it mineral, bacterial, plant, synthetic, or host product. The first group under this classification are the nonbacterial adjuvants, such as aluminum compounds. The first use of aluminum compounds as adjuvants was described in 1926. Since that time antigens precipitated with aluminum salts or antigens mixed with or adsorbed to performed aluminum compounds have been used extensively to augment immune responses in animals and humans. Aluminum compounds and similar adjuvants appear to work through the following mechanism: excretion of the antigen is slowed, thus prolonging the time of interaction between the antigen and antigen-presenting cells such as macrophages or follicular-dendritic cells. In addition, immunocompetent cells are attracted to the area of injection. Aluminum particles have been demonstrated in regional lymph nodes of rabbits seven days following immunization, and it may be that another significant function is to direct antigen to T cell-containing areas in the nodes themselves. Adjuvant potency has been shown to correlate with inflammation of the draining lymph nodes. While many studies have confirmed that antigens administered with aluminum salts led to increased humoral immunity, cell mediated immunity appears to be only slightly increased, as measured by delayed-type hypersensitivity. Aluminum hydroxide has also been described as activating the complement pathway. This mechanism may play a role in the local inflammatory response as well as immunoglobulin production and B cell memory.

Primarily because of their excellent record of safety, aluminum compounds are presently the most commonly used adjuvants in humans. They are, however, not without problems. Aluminum containing vaccines occasionally cause local reactions. Although allergic manifestations are not usually a clinical problem, aluminum compounds have been also said to attract eosinophils to the area of injection via a T cell-dependent mechanism, to induce an IgE response if injected after antigen priming, and to elicit a carrier-specific cell population with helper function for IgE response. In addition, aluminum-containing vaccines cannot be lyophilized, thus necessitating refrigerated transport and storage with the resulting risk of contamination.

Finally, and most importantly, aluminum compounds are not always successful in inducing sustained protection from disease. Thus, while aluminum salts have been a sufficient adjuvant for strong immunogens that require antibody responses only to elicit protection, they are not effective when used with weak immunogenic-like synthetic peptides of malaria for introducing cell-mediated immune responses of the type required for many infections.

Another large group of adjuvants are those of bacterial origin. Adjuvants with bacterial origins have recently been purified and synthesized (*e.g.* muramyl depeptides, lipid A) and host mediators have been cloned (Interleukin 1 and 2), providing chemically characterized products for study. The last decade has brought significant progress in the chemical purification of three adjuvants of active components of bacterial origin: *Bordetella pertussis,* lipopolysaccharide and Freund's complete adjuvant.

*B.pertussis* is of interest due to its ability to modulate cell-mediated immunity through action on T-lymphocyte populations. For lipopolysaccharide and Freund's complete adjuvant, adjuvant-active moieties have been identified and synthesized, which permit study of structure-function relationships and the possibility of modifying the original adjuvant to create a more beneficial toxic-therapeutic ratio.

Lipopolysaccharide and its various derivatives, including lipid A, have been found to be powerful adjuvants in combination with liposomes or other lipid emulsions. It is not yet certain whether derivatives with sufficiently low toxicity for use in humans can be produced. Freund's complete adjuvant is the standard in most experimental studies. However, it produces severe local and systemic inflammatory reactions which may be severe enough to cripple or kill the host. It cannot be used in humans.

Adjuvants have also been categorized by their proposed mechanisms of action. This type of classification is necessarily somewhat arbitrary because most adjuvants appear to function by more than one mechanism. Adjuvants may act through antigen localization and delivery, or by direct effects on cells making up the immune system, such as macrophages and lymphocytes. Another mechanism by which adjuvants enhance the immune response is by creation of an antigen depot. This appears to contribute to the adjuvant activity of aluminum compounds, oil emulsions, liposomes, and synthetic polymers. The adjuvant activity of lipopolysaccharides and muramyl dipeptides appears to be mainly mediated through activation of the macrophage, whereas *B. pertussis* affects both macrophages and lymphocytes. Recent and speculative approaches to immunopotentiation, such as the utilization of monokines and lymphokines, and the manipulation of the antigen, carrier, and adjuvant to augment the immune response are currently fashionable.

Small immunogens, such as the synthetic peptide of malaria, can be attached to larger proteins or other carriers to increase the immune response. The relationship between molecular size and complexity of an antigen relative to immunogenicity reflects the availability of antigenic determinants on the molecule. This relationship was first noted by Landsteiner when he demonstrated the need to complex small radicals with larger (carrier) molecules to stimulate an immune response. However, the mechanistic basis for the requirement was to await experiments that demonstrated the carrier effect and the need for a minimum of two antigenic determinants on a molecule to express immunogenicity. These determinants represented the carrier and haptenic determinants that interact with T and B lymphocytes, respectively. However, the influence of the carrier moiety extends beyond simple antigenicity through activation of T cells in T-dependent humoral responses.

The combination of determinants on an antigen molecule can influence the immune response by differential activation of helper and suppressor T cells. A model system demonstrating this effect is the genetically controlled humoral response of responder (C57B1/6) and nonresponder (DBA/1) mice to the synthetic terpolymer 1-glutamic acid⁶⁰-L-alanine³⁰-L-tyrosine¹⁰ (GAT). While C57B1/6 mice respond to this polypeptide, DBA/1 mice will respond only if the GAT is coupled to methylated bovine serum albumin (MBSA). However, if the mice are injected with GAT prior to immunization with GAT-MSBA, a detectable antibody response to GAT does not occur. The explanation for these observations is that GAT stimulates helper T cells in the responder mice but preferentially activates suppressor T cells in nonresponder mice. This predominance of suppressor cells prevents a response to GAT even when coupled to MBSA. However, if primary immunization is with GAT-MBSA, activation of helper T cells by the carrier moiety provides help that overrides the effect of any suppressor cells activated by GAT.

Determinants associated with a native protein molecule have also been demonstrated to contribute differently to help and suppression. Conjugation of an immunogenic carrier to an antigen can change the isotype of antibodies produced in response to that antigen. Purified polysaccharides from a variety of encapsulated bacteria are thymus-independent antigens due to their polymeric nature with multiple repeating antigenic determinants. While they represent protective antigens of these bacteria, the IgM antibodies produced have limited efficacy in preventing disease. Therefore, polysaccharides from *Neisseria meningitidis* and *Haemophilus influenza* type b have been conjugated to proteins, such as tetanus toxoid. These conjugated preparations act as thymus-dependent antigens and induce IgG responses to the polysaccharide moiety as well as immunologic memory. Likewise, the thymic-independent polysaccharide carriers have little potential for enhancing the immunogenicity of small peptides, such as those involved with malaria which require thymic-dependent IgG immune responses.

Publications by Feldmann and Lee state that flagella antigens of *Salmonella* organisms are typical thymic-independent antigens which stimulate strong IgM antibody responses. (See Feldmann, M, *et al.,* "The Relationship between Antigenic Structure and the Requirement for Thymus-derived cells in the Immune Response", *J.Exp.Med.,* Vol.134,pp 103-119,1971; and Lee, et al., "Decline and Spontaneous Recovery of the Monoclonal Response to Phosphorylcholine during Repeated Immunization" *J.Immun.,* Vol.113, pp 1644-1646, 1974) This published data would lead one to believe that they have little potential as adjuvants or carriers for malaria peptides or other small antigens which require thymic-dependent IgG antibody responses.

Feldman et al discloses in J. Exp. Med. (1971) 134 pp 103-119 that monomeric flagellin conjugated with donkey red cells produces a thymus-dependent antibody response. However, this reference also concludes that polymerized flagellin produces a thymus independent response which is related to the quaternary structure of the polymerized flagellin.

In Chem. Abs. (1986) vol. 105 abs No. 4730x it is described that the addition of antigenic flagellin determinants enhances the IgG response and protects mice from infection by Salmonella typhimurium.

There probably is no precise point of transition that distinguishes a carrier from an adjuvant. Obviously, the carrier moiety is contributory to a property of antigens that has been termed intrinsic adjuvanticity. The capacity of certain materials to convert a tolerogen to an immunogen has been termed as extrinsic adjuvanticity. Adjuvanticity can be enhanced by increasing the size of the antigen through aggregation of proteins or adsorption to immunogenic or inert carriers. Thus materials, such as aluminum hydroxide, latex particles, bentonite, or liposomes that adsorb antigen and enhance the immune response, are termed adjuvants. However, this observed effect of aggregation of antigen represents only a limited view of adjuvant actions which are now recognized as being extremely complex.

Small peptides and other haptens are incapable of evoking a strong immune response without the use of an adjuvant. Most adjuvants that are currently available do not evoke an immune response that is effective in protecting the animal or human against infection with the infectious agent. Thus, what is needed is a vaccine which can be administered to an animal or human and will cause the immune system to mount a prolonged and potent immune response against the peptide or other hapten that is capable of protecting the animal or human against infection.

Accordingly it is an object of the present invention to provide a vaccine that is particularly effective in providing a prolonged and potent immune response to small immunogenic determinants.

This object has been attained by a vaccine comprising 5»g-500»g per dose of a polymerized bacterial flagellin protein conjugated to a non-Salmonella antigen, wherein said antigen is chosen from the group consisting of low molecular weight peptides, polysaccharides, glycopeptides, drugs and haptens.

Additionally a method of producing this improved vaccine comprising the steps of:
a) isolating polymerized flagellin protein from a bacteria and
b) conjugating said flagella to a non-Salmonella antigen
wherein said antigen is chosen from the group consisting of low molecular weight peptides, polysaccharides, glycopeptides, drugs and haptens,
is provided.

This polymerized bacterial flagellin protein conjugated to a non-Salmonella antigen can be used for the production of a vaccine against the antigen for the immunization of a human or animal.

### Summary of the Invention

In accordance with the present invention, a vaccine that is especially effective for vaccinating a human or animal against low molecular weight peptides, polysaccharides, glycopeptides, drugs or haptens is provided. The improved vaccine provides a prolonged and potent immune response against the peptide or other small hapten.

The present invention comprises a bacterial protein conjugated to small antigenic determinants such as low molecular weight peptides, polysaccharides, glycopeptides, drugs or haptens The bacterial protein that is used in the present invention is bacterial flagella. The flagella may be derived from any flagellated mocroorganisms; however, those from *Salmonella* species are preferred. However, it is to be understood that the preferred bacterial species from which the flagella are derived for any particular application is dependent upon the particular antigen requirements of the application and is not critical for this invention. The bacterial flagella can be in the native polymerized form or can be repolymerized flagellin.

The present invention also includes combination of the conjugated flagella and antigen with an adjuvant, such as a block copolymer. The preferred adjuvant that can be used with the vaccine of the present invention is a block copolymer that comprises a polymer of hydrophilic polyoxyethylene (POE) built on an ethylene diamine initiator. Polymers of hydrophobic polyoxypropylene (POP) are then added to block of hydrophilic polyoxyethylene (POE). This results in an octablock copolymer with the following general formula: 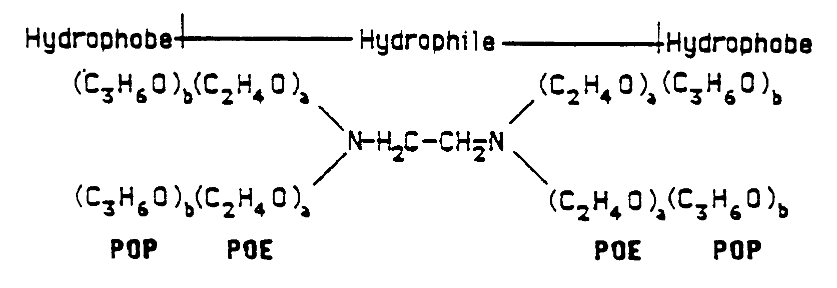
wherein:
a is a number such that the hydrophile portion represented by polyoxyethylene (C₂H₄O)ₐ (POE) constitutes between 10% to 40% of the total molecular weight of the compound;
the mean aggregate molecular weight of the hydrophobe portion of the octablock copolymer consisting of polyoxypropylene (C₃H₆O)_{b} (POP) is between approximately 4000 and 9000 daltons; and
b is a number such that the polyoxypropylene (C₃H₆O)_{b} (POP) portion of the total molecular weight of the octablock copolymer constitutes between approximately 60% and 90% of the compound.

Flagella can be used as a very effective adjuvant and carrier for inducing antibody responses which are long-lasting, high titer, and of high avidity against small antigenic determinants, such as low molecular weight peptides, polysaccharides, glycopeptides, drugs and haptens. The low molecular weight peptides can be either synthetic or genetically engineered. Examples of a genetically engineered peptides are those currently available for malaria. The improved vaccine comprising a conjugate of a small antigenic determinant and flagella can be used to induce strong and prolonged thymic-dependent IgG antibody responses.

An advantage of the present invention is that an effective vaccine that can utilize a synthetic peptide, such as malaria, to produce a sustained immune response capable of protecting an individual from infection by the malaria parasite. is provided.

An application of the present invention might lead to an effective vaccine that can utilize a synthetic peptide of the AIDS virus to produce an immune response that is effective in preventing the disease.

Yet another advantage of the present invention is that a vaccine that is capable of stimulating the immune system of an animal or human to produce a potent and prolonged IgG response to a small immunogenic determinant, such as a peptide or hapten. is provided

Another advantage of the present invention is that the vaccine has very low toxicity for humans or animals.

Yet another advantage of the present invention is that a vaccine which cases little or no local allergic reaction. is provided.

A further advantage of the present invention is that a vaccine which can be lyophilized. is provided.

Another advantage of the present invention is that an adjuvant that can be used with a vaccine preparation. is provided

### Brief Description of the Drawings

Fig. 1 is a graph illustrating the antibody titer in a mouse immunized with trinitrophenol (TNP) conjugated to flagella protein from *Salmonella.*

Fig. 2 is a graph illustrating the dose response of a mouse immunized with TNP conjugated to flagella protein from *Salmonella.*

Fig. 3 is a graph comparing the immune response of a mouse immunized with TNP conjugated to hen egg albumin (hEA) and TNP conjugated to flagella protein from *Salmonella.* The graph also compares using the two compounds with and without the adjuvant Polyphore 32:5 (CytRx Corporation, Atlanta, Georgia).

### Detailed Description

The present invention comprises a vaccine that is especially useful for immunizing an animal or human against low molecular weight peptides, polysaccharides, glycopeptides, drugs or haptens. According to the present invention, the low molecular weight peptides polysaccarides, glycopeptides, drugs or haptens are conjugated to flagella that is derived from a microorganism. The flagella may be derived from an flagellated microorganism; however, those from *Salmonella* species are preferred. It is to be understood that the preferred bacterial species from which the flagella are derived for any particular application is dependent upon the particular antigen requirements of the application and is not critical for this invention.

Some bacteria possess a single flagellum while others have a tuft of flagella and still others have flagella distributed over the entire cell surface. Bacterial flagella are between 10 and 35 nm in diameter and may sometimes exceed 10 to 15 »m in length, or many times the diameter of the cell. Most bacterial flagella show a regular and uniform curl with a wavelength of about 2.5 »m.

When bacterial flagella, which are protein in nature, are acidified to pH3, they dissociate into identical monomeric subunits called flagellin, which has a molecular weight of approximately 40,000 in most species. Under appropriate conditions of pH and salt concentration, flagellin monomers will spontaneously reaggregate to form structures that appear to be identical with intact flagella possessing periodic curls of the same wavelength as the native flagella.

Intact bacterial flagella in the native form or fixed with a number of fixative agents can be used in practicing the present invention. Additionally, repolymerized flagellin is satisfactory in practicing the present invention. It is believed that an essential component of the present invention is that the preparation consists of a polymer composed of flagellin molecules regularly spaced in a geometric pattern to produce the elongated flagellar structure typical of the particular microorganism.

Antigens are compounds which, when introduced into a mammal, will result in the formation of antibodies. Representative of the antigens that can be used according to the present invention are low molecular weight peptides, polysaccharides, glycopeptides, drugs and haptens
Haptens are compounds which, when bound to an immunogenic carrier and introduced into a chordate, will elicit formation of antibodies specific for the hapten. Representative of the haptens are steroids such as estrogens and cortisones, low molecular weight peptides, other low molecular weight biological compounds, drugs such as antibiotics and chemotherapeutic compounds, industrial pollutants, flavoring agents, food additives, and food contaminants, and/or their metabolites or derivatives.

A number of procedures for preparing flagella from bacterial cultures have been developed and are well-known to those of ordinary skill in the art. The preferred procedure is a modification of the procedure of Kobayashi, Rinker, and Koffler *Arch. Biochem. Biophys.* 84, 342-362 (1959) as described herein.

*Salmonella typhi* organisms of strain of TY2 are grown in motility agar. The highly motile organisms should be selected because they produced the most flagella. Organisms are then inoculated in 20 liters of trypticase soy broth and incubated at 37°C for approximately 30 hours until the end of the log phase of growth. The organisms may be killed at this time by the addition of formaldehyde to produce a 0.3% suspension. The organisms are preferably collected by centrifugation; however, care should be taken to avoid production of excessive shear force. The flagella are then removed from the organisms by shaking vigorously for 20 minutes in a shaker. Other mixes and devices which produce a shear force to break off the flagella without disrupting the organism are equally satisfactory.

The flagella are then separated from the cell bodies by differential centrifugation. The cell bodies are removed by centrifuging at approximately 2000 rpm in a standard laboratory centrifuge. The flagella are then collected by ultracentrifugation at 30,000 rpm. The flagella are then resuspended and recentrifuged in an ultracentrifuge, and soluble contaminating materials are poured off. Large contaminating materials will form a black spot at the bottom of the transparent flagella pellet. This material is physically removed and discarded. The end product derived from 20 liters of bacterial culture will be approximately 100 mg of purified flagella.

Flagellin may be produced by acidifying unfixed flagella at a pH of approximately 2 overnight. This treatment disassociates the flagellar proteins to produce the monomers of flagellin which have a molecular weight of approximately 30,000. The monomers reassemble into the polymerized flagella when allowed to stand at neutral pH for a period of at least 24 hours. The repolymerized flagellin is nearly as effective as the native flagella as an adjuvant and carrier for small antigen moieties. The monomeric flagellin or proteolytic cleavage fragments of flagellin protein are very much less effective.

The antigen moieties can be chemically conjugated to the flagella by any one of the standard means well-known to those of ordinary skill in the art. One of the simplest and most effective means is by using gluteraldehyde. Gluteraldehyde is a divalent cross-linking compound which covalently attaches the antigen to the flagella and further fixes the flagella preparation. Other chemical cross-linking reagents or chemical antigen derivatives, such as dinitrofluorobenzene, are effective.

The amounts of antigen attached to the flagella varies with the particular application and is not a critical component of this invention. Preferably, between 2 and 10 peptide or hapten units per flagellin monomer in the flagella preparation is sufficient.

The conjugated flagella preparation is purified by dialysis, centrifugation, or any other standard method. The material is then resuspended in saline at a concentration approximating 100 »g/ml. This preparation is effective in low doses between 1 and 100 »g per injection. A dose of 10 »g produces a satisfactory response in many situations. The material can be injected by any convenient route, intravenous, subcutaneous, intramuscular, or intraperitoneal. The subcutaneous or intramuscular route is usually the most convenient for many vaccine purposes.

As an example, injections of 20 »g of *Salmonella typhi* flagella conjugated with dinitrophenol resulted in IgG antibody titers specific for the hapten DNP which rose at the end of the first week after injection and persisted for several months.

Persistence of the immune response to flagella and to antigenic moieties conjugated to flagella is unusual and unexpected. The material does not form a local depot of antigen at the site of injection. 90 to 95% of the injected dose of flagella is broken down and excreted within 24 hours. A portion of the material is retained for a prolonged time in germinal centers within local lymph nodes. It is believed that the presence of this antigen in germinal centers is responsible for the prolonged antibody production.

This invention has numerous advantages over other available adjuvant preparations. It produces very little inflammation at the site of injection and is entirely biodegradable. This contrasts sharply with oil emulsions or mineral salts, such as aluminum. Very small doses of antigen are required to produce prolonged immmune responses. A significant portion of the antibody is complement-fixing IgG which is the type required for protection against malaria, sporozoites, and other important infections. The product is stable especially when prepared with fixatives, such as gluteraldehyde. It can be lyophilized and stored at room temperature indefinitely. When reconstituted with saline, it is stable for several weeks with refrigeration and several days at room temperature.

Unlike live attenuated vaccines which may produce infections in susceptible hosts, this vaccine preparation consists only of polymerized protein with traces of polysaccharide.

The dose of a vaccine prepared according to the present invention is between 5»g ad 500»g. The optimal dose for any vaccine will depend upon the antigen that is conjugated with the flagella protein and the immunological condition of the animal or human that is being vaccinated.

The vaccine of the present invention also includes the administration of the vaccine with an adjuvant to further enhance the immune response. The preferred adjuvant (Polyphore™ 32:5, CytRx Corporation, Atlanta, Georgia) that can be used with the vaccine of the present invention is a block copolymer that comprises a polymer of hydrophilic polyoxyethylene (POE) built on a ethylene diamine initiator. Polymers of hydrophobic polyoxypropylene (POP) are then added to a block of hydrophilic polyoxyethylene (POE). This results in an octablock copolymer with the following general formula: 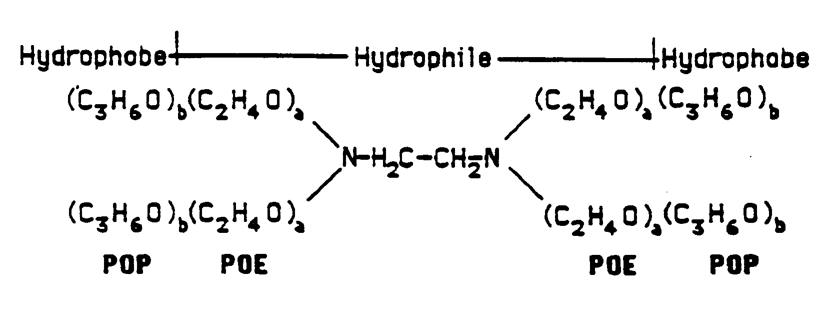
wherein:
a is a number such that the hydrophile portion represented by polyoxyethylene (C₂H₄O)ₐ (POE) constitutes between 10% to 40% of the total molecular weight of the compound;
the mean aggregate molecular weight of the hydrophobe portion of the octablock copolymer consisting of polyoxypropylene (C₃H₆O)_{b} (POP) is between 4000 and 9000 daltons; and
b is a number such that the polyoxypropylene (C₃H₆O)_{b} (POP) portion of the total molecular weight of the octablock copolymer constitutes between 60% and 90% of the compound.

The preferred adjuvant has the following formula: 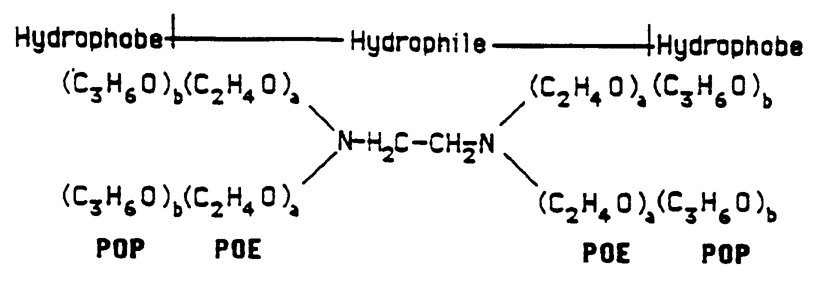
wherein a is equal to approximately 5 and b is equal to approximately 32.

Another copolymer that can be used with the vaccine comprising the present invention has the following formula:

HO(C₂H₄O)ₐ(C₃H₆O)_{b}(C₂H₄O)ₐH

wherein the molecular weight of the hydrophobe (C₃H₆O) is between 2000 to 5000 and the total molecular weight of the compound is between 2300 and 6000 (CytRx Corporation, Atlanta, Georgia).

The preferred adjuvant has the following formula:

HO(C₂H₄O)ₐ(C₃H₆O)_{b} (C₂H₄O)ₐH

wherein the molecular weight of the hydrophobe (C₃H₆O) is approximately 4300 and the percentage of hydrophile (C₂H₄O)ₐ is approximately 10% by weight. (CytRx Corporation, Atlanta, Georgia).

The polymer blocks are formed by condensation of ethylene oxide and propylene oxide onto a tetrafunctional ethylene diamine initiator at elevated temperature and pressure in the presence of a basic catalyst. There is some statistical variation in the number of monomer units which combine to form a polymer chain in each copolymer. The molecular weights given are approximations of the average weight of copolymer molecule in each preparation. A further description of the preparation of these block copolymers is found in U S-A-2,674,619 and U S-A-2,979,528 which are incorporated herein by reference. (Also see "A Review of Block Polymer Surfactants", Schmolka, I.R., *J. Am Oil Chemists' Soc.,* 54:110-116 (1977) and *Block and Graft Copolymerization,* Volume 2 edited by R.J. Ceresa, John Wiley & Sons, New York (1976) which are incorporated herein by reference.)

The vaccine which comprises the present invention is mixed with the octablock copolymer and administered to the human or animal. The preferred amount of adjuvant administered with the vaccine of the present invention is between 0.1 mg and 5.0 mg with the most preferred amount between 0.5 mg and 2 mg.

The following specific examples will illustrate the invention as it applies to enhancing the immune response of an organism to small haptens.

### Example I

*Salmonella typhi* organisms of strain of TY2 are grown in motility agar. Organisms are then inoculated in 20 liters of trypticase soy broth and incubated at 37° for 30 hours until the end of the log phase of growth. The organisms are killed at this time by the addition of formaldehyde to produce a 0.3% suspension. The organisms are collected by centrifugation. Care should be taken to avoid production of excessive shear force. The flagella are then removed from the organisms by shaking vigorously for 20 minutes in a shaker. Other mixes and devices which produce a shear force to break off the flagella without disrupting the organism are equally satisfactory.

The flagella are then separated from the cell bodies by differential centrifugation. The cell bodies are removed by centrifuging at 2000 rpm in a standard laboratory centrifuge. The flagella are then collected by ultracentrifugation at 30,000 rpm. After the ultracentrifugation, the flagella are resuspended and recentrifuged in an ultracentrifuge, and soluble contaminating materials are poured off. Large contaminating materials form a black spot at the bottom of the transparent flagella pellet. This material is physically removed and discarded. The end product derived from 20 liters of bacterial culture is approximately 100 mg or purified flagella.

### Example II

Flagellin is produced by acidifying the flagella at a pH of approximately 2 for 12 hours. This treatment disassociates the flagellar proteins to produce the three monomers of flagellin which have a molecular weight of approximately 30,000. The monomers reassemble into the polymerized flagella when allowed to stand at neutral pH for a period of at least 24 hours.

### Example III

Gluteraldehyde is a divalent cross-linking compound which covalently attaches the peptide to the flagella and further fixes the flagella preparation. These methods of conjugating a functional group to a protein are well-known to one of ordinary skill in the art. Other chemical cross-linking reagents or chemical antigen derivatives, such as dinitrofluorobenzene are effective.

### Example IV

The conjugated flagella preparation is purified by dialysis, centrifugation, or any other standard method. The material is then resuspended in saline at a concentration approximating 100 »g/ml. This preparation is effective in low doses between 1 and 100 »g per injection. A dose of 10 »g produces a satisfactory response in many situations. The material can be injected by any convenient route, intravenous, subcutaneous, intramuscular, or intraperitoneal. The subcutaneous or intramuscular route is usually the most convenient for many vaccine purposes.

### Example V

An ELISA assay is used for the determination of antibody directed against the trinitrophenol hapten. It is a modification of the method originally published by Saunders (See Saunders, G.C., "'The art of solid phase enzyme immunoassay including selected protocols". in: *Immunassays in the Clinical Laboratory,* Alan R. Liss, New York, pp. 111-112, 1979).

The assay uses a protein, bovine serum albumin, hydrogel to reduce denaturation of proteins adherent to the plastic support and the use of proteins and surfactants to reduce non-specific adsorption of proteins which tend to increase background and reduce sensitivity. Glutaraldehyde is used to attach antigen to BSA-coated 96-well microtiter plates. Unbound glutaraldehyde is washed off. Antigen added to the plates attaches to the plate covalently via the free aldehyde groups of gluteraldehyde.

Remaining aldehyde groups are blocked with lysine and the plate is ready to use. The plates are incubated with various dilutions of antiserum, washed and then a second antibody such as peroxidase-conjugated goat anti-mouse IgG or one of the subclasses. The plates are washed and substrate (e.g., orthophenylene diamine with peroxide) is added. The resulting absorbance at 492 nm is read by a Titertek Multiscan photometer. The titer of antibody is calculated as the dilution of antiserum required to produce a 1/3 to 1/2 maximal O.D. of the background. This is normalized by comparison to a reference antiserum simultaneously with the sample. This facilitates comparison of titers run on different days. The relative avidity of antibodies in relation to one another is estimated by analysis of the slope of the curve of O.D. versus serum dilution.

### Example VI

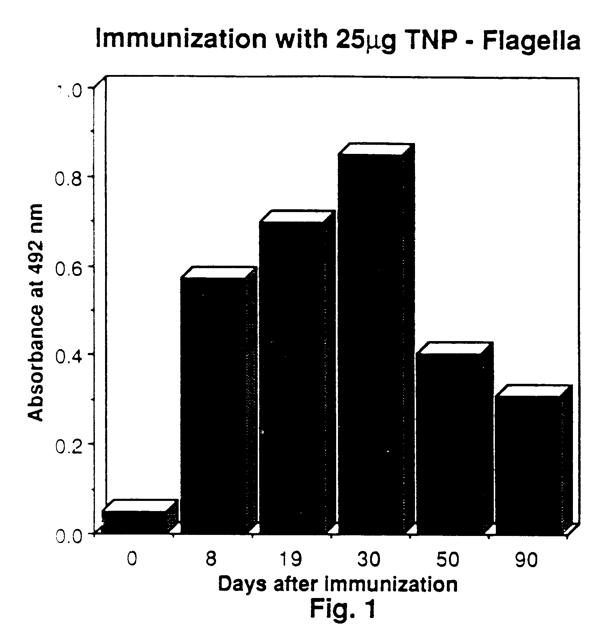

In the following experiment, 25 »g of flagella conjugated with an average of 4 TNP molecules per flagella is administered to mice via a hind footpad. The TNP-conjugated flagella was administered in a volume of 0.5 ml of saline. Antibody specific for TNP is measured at the following times after administration of the TNP-conjugated flagella: 8 days, 19 days, 30 days, 50 days and 90 days. The results of this experiment are shown in Fig. 1. As can be seen, the immune response to the TNP-conjugated flagella is still significantly high even after 90 days. The response to conventional TNP conjugates, such as TNP-conjugated hen egg albumin is much shorter in duration and the antibody titers are much lower. Animals frequently do not respond at all with detectable antibody to a hapten on a soluble protein carrier after a single injection.

### Example VII

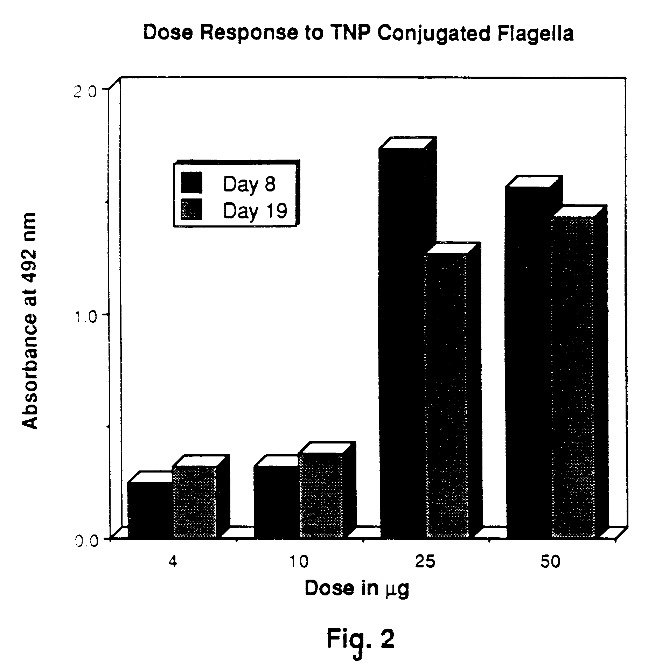

The dose response of a mouse is measured by administering varying doses of TNP-conjugated flagella. Flagella conjugated with an average of 4 TNP molecules per flagellin molecule (molecular weight approximately 40,000) is administered to mice via a hind footpad. The TNP-conjugated flagella is administered in a volume of 0.5 ml of saline. The following concentrations of TNP-conjugated flagella are administered to mice: 4 »g, 10»g, 25»g and 50»g. The antibody produced in response to the TNP-conjugated flagella is measured 8 days and 19 days after administration of the TNP-conjugated flagella. The results of this experiment is shown in Fig. 2.

### Example VIII

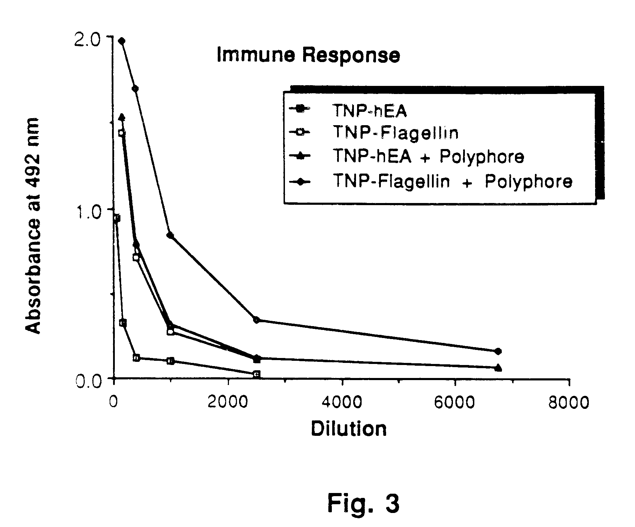

A comparison of the immunologic response of mice to TNP conjugated to hen egg albumin (hEA) and TNP conjugated to bacteria flagella protein is shown in Fig. 3. In this experiment, TNP is conjugated to hEA using the reactive derivative trinitrobenzene sulfonic acid (TNBS) in the same fashion as flagella. 100 »g of the TNP-conjugated hEA or 25 »g of TNP-conjugated flagella are administered to mice via a hind footpad. Ten days after administration of the TNP-conjugated proteins, antibody titer is measured according to Example V. As shown in Fig. 3, the TNP-conjugated flagella induced a significantly greater immune response, as measured by antibody titer, than did the TNP-conjugated hEA. It should be noted that the amount of TNP-hEA administered in this experiment was four times the amount of TNP-conjugated flagella (100»g of TNP-hEA vs 25»g of TNP-conjugated flagella).

### Example IX

The same preparations used in Example IX are administered to mice with the addition of 1.0 mg of Polyphore™ 32:5 adjuvant (CytRx Corporation, Atlanta, GA). 100 »g of the TNP-conjugated hEA or 25 »g of TNP-conjugated flagella are administered to mice via a hind footpad. Ten days after administration of the TNP-conjugated proteins with the adjuvant, antibody titer is measured according to Example V. The results of these experiments are summarized in Fig. 3. As shown, the adjuvant raised the immune response to both the TNP-conjugated hEA and the TNP-conjugated flagella. However, the TNP-conjugated flagella induced a significantly greater immune response than did the TNP-conjugated hEA.

## Claims

1. A vaccine comprising 5»g-500»g per dose of a polymerized bacterial flagellin protein conjugated to a non-Salmonella antigen, wherein said antigen is chosen from the group consisting of low molecular weight peptides, polysaccharides, glycopeptides, drugs and haptens.

2. The vaccine of claim 1 wherein the bacterial flagella is isolated from Salmonella species.

3. The vaccine of claim 2 wherein the Salmonella species is Salmonella typhi.

4. The vaccine of claim 1 wherein the vaccine is combined with an adjuvant.

5. The vaccine of claim 4 wherein the adjuvant has the following formula 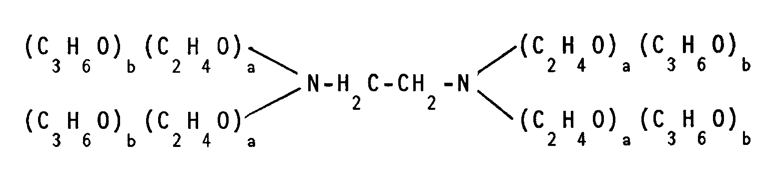 wherein
a is a number such that the hydrophile portion represented by polyoxyethylene (C₂H₄O)ₐ (POE) constitutes between 10% and 40% of the total molecular weight of the compound,
the mean aggregate molecular weight of the hydrophobe portion of the octablock copolymer consisting of polyoxypropylene (C₃H₆O)_{b} (POP) is between 4000 and 9000 daltons, and
b is a number such that the polyoxypropylene (C₃H₆O)_{b} (POP) portion of the total molecular weight of the octablock copolymer constitutes between 60% and 90% of the compound.

6. The vaccine of claim 4, wherein the adjuvant has the following formula 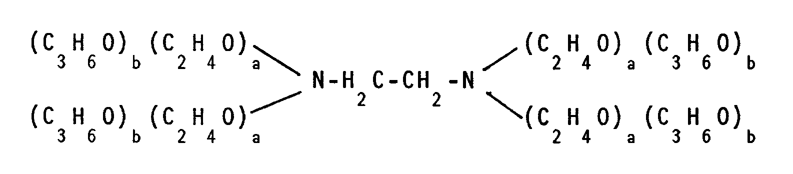 wherein a is equal to 5 and b is equal to 32.

7. The vaccine of claim 4, wherein the adjuvant has the following formula
HO(C₂H₄O)ₐ (C₃H₆O)_{b} (C₂H₄O)ₐH
wherein the molecular weight of the hydrophobe (C₃H₆O) is between 2000 to 5000 and the total molecular weight of the compound is between 2300 and 6000.

8. The vaccine of claim 4, wherein the adjuvant has the following formula
HO(C₂H₄O)ₐ (C₃H₆O)_{b} (C₂H₄O)ₐH
wherein the molecular weight of the hydrophobe (C₃H₆O) is approximately 4300 and the percentage of hydrophile (C₂H₄O)ₐ is approximately 10% by weight.

9. A method of producing an improved vaccine comprising
(a) isolating polymerized flagellin protein from a bacteria, and
(b) conjugating said flagella to a non-Salmonella antigen, wherein said antigen is chosen from the group consisting of low molecular weight peptides, polysaccharides, glycopeptides, drugs and haptens.

10. The method of claim 9, wherein the vaccine is combined with an adjuvant.

11. The method of claim 10, wherein the adjuvant has the following formula 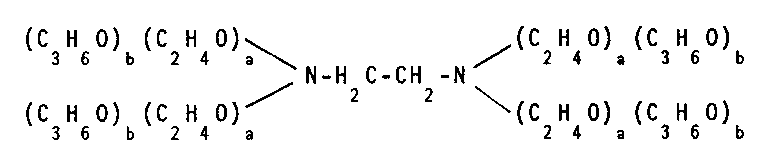 wherein
a is a number such that the hydrophile portion represented by polyoxyethylene (C₂H₄O)ₐ (POE) constitutes between 10% and 40% of the total molecular weight of the compound,
the mean aggregate molecular weight of the hydrophobe portion of the octablock copolymer consisting of polyoxypropylene (C₃H₆O)_{b} (POP) is between 4000 and 9000 daltons, and
b is a number such that the polyoxypropylene (C₃H₆O)_{b} (POP) portion of the total molecular weight of the octablock copolymer constitutes between 60% and 90% of the compound.

12. The method of claim 10, wherein the adjuvant has the following formula 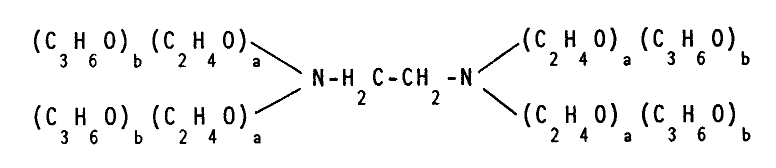 wherein a is equal to 5 and b is equal to 32.

13. The method of claim 10, wherein the adjuvant has the following formula
HO(C₂H₄O)ₐ(C₃H₆O)_{b}(C₂H₄O)ₐH
wherein the molecular weight of the hydrophobe (C₃H₆O) is between 2000 to 5000 and the total molecular weight of the compound is between 2300 and 6000.

14. The method of claim 10, wherein the adjuvant has the following formula
HO(C₂H₄O)ₐ (C₃H₆O)_{b} (C₂H₄O)ₐH
wherein the molecular weight of the hydrophobe (C₃H₆O) is approximately 4300 and the percentage of hydrophile (C₂H₄O)ₐ is approximately 10% by weight.

15. Use of a polymerized bacterial flagellin protein conjugated to a non-Salmonella antigen for the production of a vaccine against the antigen for the immunization of a human or animal, wherein said antigen is chosen from the group consisting of low molecular weight peptides, polysaccharides, glycopeptides, drugs and haptens.

16. The use of claim 15, wherein the vaccine is combined with an adjuvant.

17. The use of claim 16, wherein the adjuvant has the following formula 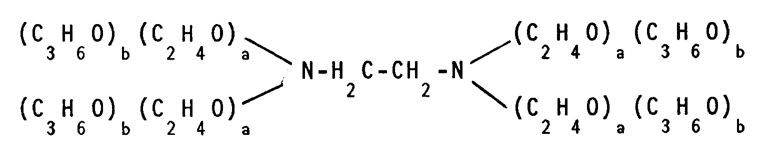 wherein
a is a number such that the hydrophile portion represented by polyoxyethylene (C₂H₄O)ₐ (POE) constitutes between 10% and 40% of the total molecular weight of the compound,
the mean aggregate molecular weight of the hydrophobe portion of the octablock copolymer consisting of polyoxypropylene (C₃H₆O)_{b} (POP) is between 4000 and 9000 daltons, and
b is a number such that the polyoxypropylene (C₃H₆O)_{b} (POP) portion of the total molecular weight of the octablock copolymer constitutes between 60% and 90% of the compound.

18. The use of claim 16, wherein the adjuvant has the following formula 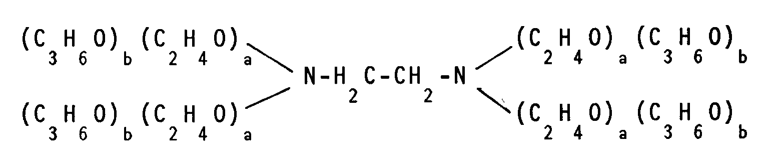 wherein a is equal to 5 and b is equal to 32.

19. The use of claim 16, wherein the adjuvant has the following formula
HO(C₂H₄O)ₐ(C₃H₆O)_{b}(C₂H₄O)ₐH
wherein the molecular weight of the hydrophobe (C₃H₆O) is between 2000 to 5000 and the total molecular weight of the compound is between 2300 and 6000.

20. The use of claim 16, wherein the adjuvant has the following formula
HO(C₂H₄O)ₐ(C₃H₆O)_{b}(C₂H₄O)ₐH
wherein the molecular weight of the hydrophobe (C₃H₆O) is approximately 4300 and the percentage of hydrophile (C₂H₄O)ₐ is approximately 10% by weight.

## Patentansprüche

1. Vakzine, enthaltend 5»g-500»g pro Dosis eines polymerisierten Proteins von bacteria flagella, konjugiert mit einem Nicht-Salmonella-Antigen, wobei das Antigen aus der aus Peptiden mit niedrigem Molekulargewicht, Polysacchariden, Glycopeptiden, Arzneimitteln und Haptenen bestehenden Gruppe ausgewählt ist.

2. Vakzine nach Anspruch 1,
**dadurch gekennzeichnet,**
daß die bacteria flagella aus Salmonellenspezies isoliert sind.

3. Vakzine nach Anspruch 2,
**dadurch gekennzeichnet,**
daß die Salmonellenspezies Salmonella typhi sind.

4. Vakzine nach Anspruch 1,
**dadurch gekennzeichnet,**
daß der Impfstoff mit einem Hilfsstoff kombiniert ist.

5. Vakzine nach Anspruch 4,
**dadurch gekennzeichnet,**
daß der Hilfsstoff der folgenden Formel genügt 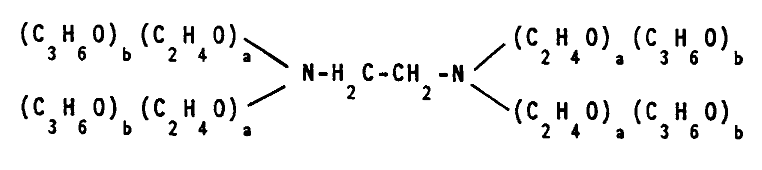 in der a eine solche Zahl ist, daß der hydrophile Teil, wiedergegeben durch Polyoxyethylen (C₂H₄O)ₐ (POE), zwischen 10% and 40% des gesamten Molekulargewichtes der Verbindung ausmacht und das mittlere Aggregatmolekulargewicht des hydrophoben Teils des Octablockcopolymeren, bestehend aus Polyoxypropylen (C₃H₆O)_{b} (POP), zwischen 4000 and 9000 Daltons beträgt und b eine solche Zahl ist, daß der Polyoxypropylenanteil (C₃H₆O)_{b} (POP) des gesamten Molekulargewichts des Octablockcopolymeren zwischen 60% and 90% der Verbindung ausmacht.

6. Vakzine nach Anspruch 4,
**dadurch gekennzeichnet,**
daß der Hilfsstoff der folgenden Formel genügt 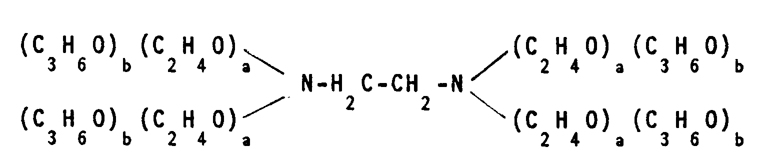 in der a gleich 5 und b gleich 32 ist.

7. Vakzine nach Anspruch 4,
**dadurch gekennzeichnet,**
daß der Hilfsstoff der folgenden Formel genügt
HO(C₂H₄O)ₐ(C₃H₆O)_{b}(C₂H₄O)ₐH
in der das Molekulargewicht des hydrophoben Teils (C₃H₆O) zwischen 2000 und 5000 beträgt und das Gesamtmolekulargewicht der Verbindung zwischen 2300 und 6000 liegt.

8. Vakzine nach Anspruch 4,
**dadurch gekennzeichnet,**
daß der Hilfsstoff der folgenden Formel genügt
HO(C₂H₄O)ₐ(C₃H₆O)_{b}(C₂H₄O)ₐH
in der das Molekulargewicht des hydrophoben Teils (C₃H₆O) etwa 4300 beträgt und der Prozentsatz des hydrophilen Teils (C₂H₄O)ₐ etwa 10 Gew.-% beträgt.

9. Verfahren zum Herstellen einer verbesserten Vakzine durch
(a) Isolieren des polymerisierten Proteins von bacteria flagella aus Bakterien und
(b) Konjugieren der Flagella mit einem Nicht-Salmonellen-Antigen, wobei das Antigen aus der aus Peptiden mit niedrigem Molekulargewicht, Polysacchariden, Glycopeptiden, Arzneimitteln und Haptenen bestehenden Gruppe ausgewählt ist.

10. Verfahren nach Anspruch 9,
**dadurch gekennzeichnet,**
daß der Impfstoff mit einem Hilfsstoff kombiniert ist.

11. Verfahren nach Anspruch 10,
**dadurch gekennzeichnet,**
daß der Hilfsstoff der folgenden Formel genügt 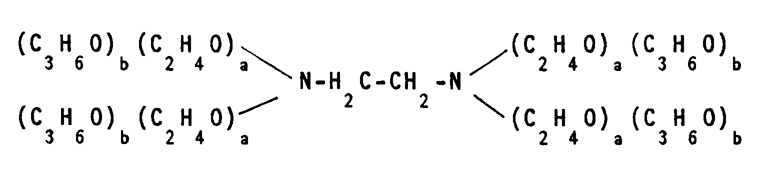 in der a eine solche Zahl ist, daß der hydrophile Teil, wiedergegeben durch Polyoxyethylen (C₂H₄O)ₐ (POE), zwischen 10% and 40% des gesamten Molekulargewichtes der Verbindung ausmacht und das mittlere Aggregatmolekulargewicht des hydrophoben Teils des Octablockcopolymeren, bestehend aus Polyoxypropylen (C₃H₆O)_{b} (POP), zwischen 4000 and 9000 Daltons beträgt und b eine solche Zahl ist, daß der Polyoxypropylenanteil (C₃H₆O)_{b} (POP) des gesamten Molekulargewichts des Octablockcopolymeren zwischen 60% and 90% der Verbindung ausmacht.

12. Verfahren nach Anspruch 10,
**dadurch gekennzeichnet,**
daß der Hilfsstoff der folgenden Formel genügt 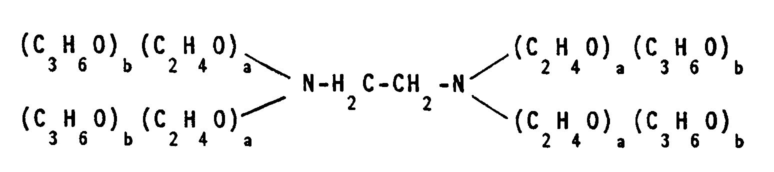 in der a gleich 5 und b gleich 32 ist.

13. Verfahren nach Anspruch 10,
**dadurch gekennzeichnet,**
daß der Hilfsstoff der folgenden Formel genügt
HO(C₂H₄O)ₐ(C₃H₆O) _{b}(C₂H₄O)ₐH
in der das Molekulargewicht des hydrophoben Teils (C₃H₆O) zwischen 2000 und 5000 beträgt und das Gesamtmolekulargewicht der Verbindung zwischen 2300 und 6000 liegt.

14. Verfahren nach Anspruch 10,
**dadurch gekennzeichnet,**
daß der Hilfsstoff der folgenden Formel genügt
HO(C₂H₄O)ₐ(C₃H₆O)_{b}(C₂H₄O)ₐH
in der das Molekulargewicht des hydrophoben Teils (C₃H₆O) etwa 4300 beträgt und der Prozentsatz des hydrophilen Teils (C₂H₄O)ₐ etwa 10 Gew.-% beträgt.

15. Verwendung eines polymerisierten Proteins von bacteria flagella, konjugiert mit einem Nicht-Salmonella-Antigen zur Herstellung eines Impfstoffes gegen das Antigen zur Immunisierung eines Menschen oder Tieres, wobei das Antigen aus der aus Peptiden mit niedrigem Molekulargewicht, Polysacchariden, Glycopeptiden, Arzneimitteln und Haptenen bestehenden Gruppe ausgewählt ist.

16. Verwendung nach Anspruch 15,
**dadurch gekennzeichnet,**
daß der Impfstoff mit einem Hilfsstoff kombiniert ist.

17. Verwendung nach Anspruch 16,
**dadurch gekennzeichnet,**
daß der Hilfsstoff der folgenden Formel genügt 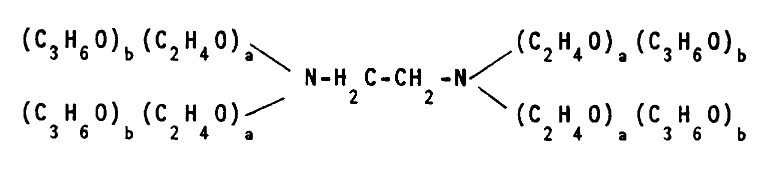 in der a eine solche Zahl ist, daß der hydrophile Teil, wiedergegeben durch Polyoxyethylen (C₂H₄O)ₐ (POE), zwischen 10% and 40% des gesamten Molekulargewichtes der Verbindung ausmacht und das mittlere Aggregatmolekulargewicht des hydrophoben Teils des Octablockcopolymeren, bestehend aus Polyoxypropylen (C₃H₆O)_{b} (POP), zwischen 4000 and 9000 Daltons beträgt und b eine solche Zahl ist, daß der Polyoxypropylenanteil (C₃H₆O)_{b} (POP) des gesamten Molekulargewichts des Octablockcopolymeren zwischen 60% and 90% der Verbindung ausmacht.

18. Verwendung nach Anspruch 16,
**dadurch gekennzeichnet,**
daß der Hilfsstoff der folgenden Formel genügt 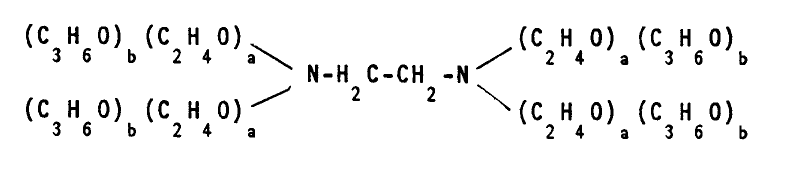 in der a gleich 5 und b gleich 32 ist.

19. Verwendung nach Anspruch 16,
**dadurch gekennzeichnet,**
daß der Hilfsstoff der folgenden Formel genügt
HO(C₂H₄O)ₐ(C₃H₆O)_{b}(C₂H₄O)ₐH
in der das Molekulargewicht des hydrophoben Teils (C₃H₆O) zwischen 2000 und 5000 beträgt und das Gesamtmolekulargewicht der Verbindung zwischen 2300 und 6000 liegt.

20. Verwendung nach Anspruch 16,
**dadurch gekennzeichnet,**
daß der Hilfsstoff der folgenden Formel genügt
HO(C₂H₄O)ₐ(C₃H₆O)_{b}(C₂H₄O)ₐH
in der das Molekulargewicht des hydrophoben Teils (C₃H₆O) etwa 4300 beträgt und der Prozentsatz des hydrophilen Teils (C₂H₄O)ₐ etwa 10 Gew.-% beträgt.

## Revendications

1. Vaccin comprenant 5 »g à 500 »g par dose d'une protéine de flagelline bactérienne polymérisée conjuguée à un antigène non salmonella, où l'antigène est choisi parmi le groupe consistant en des peptides, des polysaccharides, des glycopeptides, des médicaments et des haptènes de poids moléculaire faible.

2. Vaccin suivant la revendication 1, où les flagelles bactériens sont isolés d'espèces de salmonella.

3. Vaccin suivant la revendication 2, où l'espèce de salmonella est Salmonella typhi.

4. Vaccin suivant la revendication 1, où le vaccin est combiné à un adjuvant.

5. Vaccin suivant la revendication 4, où l'adjuvant a la formule suivante : 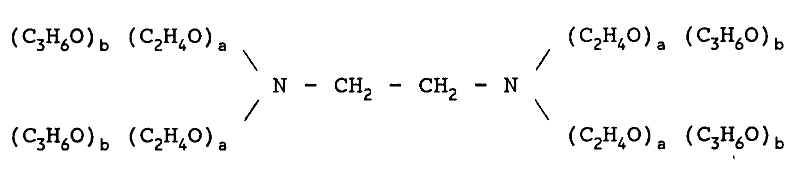 où :
a est un nombre tel que la partie hydrophile représentée par le polyoxyéthylène (C₂H₄O)ₐ (POE) constitue entre 10% et 40% du poids moléculaire total du composé;
le poids moléculaire de l'agrégat moyen de la partie hydrophobe du copolymère octaséquencé consistant en du polyoxypropylène (C₃H₆O)_{b} (POP) se situe entre 4000 et 9000 daltons, et
b est un nombre tel que la partie polyoxypropylène (C₃H₆O)_{b} (POP) du composé constitue entre 60% et 90% du poids moléculaire total du copolymère octaséquencé.

6. Vaccin suivant la revendication 4, où l'adjuvant a la formule suivante : 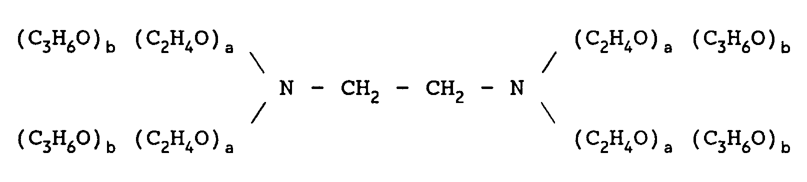 où, a est égal à 5 et b est égal à 32.

7. Vaccin suivant la revendication 4, où l'adjuvant a la formule suivante :
HO(C₂H₄O)ₐ (C₃H₆O)_{b} (C₂H₄O)ₐ H
où le poids moléculaire du (C₃H₆O) hydrophobe se situe entre 2000 et 5000 et le poids moléculaire total du composé se situe entre 2300 et 6000.

8. Vaccin suivant la revendication 4, où l'adjuvant a la formule suivante :
HO(C₂H₄O)ₐ (C₃H₆O)_{b} (C₂H₄O)ₐ H
où, le poids moléculaire du (C₃H₆O) hydrophobe se situe à environ 4300 et le pourcentage de (C₂H₄O)ₐ hydrophile est d'environ 10% en poids.

9. Procédé de production d'un vaccin amélioré comprenant :
(a) l'isolement de la protéine de flagelline polymérisée d'une bactérie, et
(b) la conjugaison des flagelles à un antigène non salmonella, où l'antigène est choisi parmi le groupe consistant en des peptides, des polysaccharides, des glycopeptides, des médicaments et des haptènes de poids moléculaire faible.

10. Procédé suivant la revendication 9, où le vaccin est combiné à un adjuvant.

11. Procédé suivant la revendication 10, où l'adjuvant a la formule suivante : 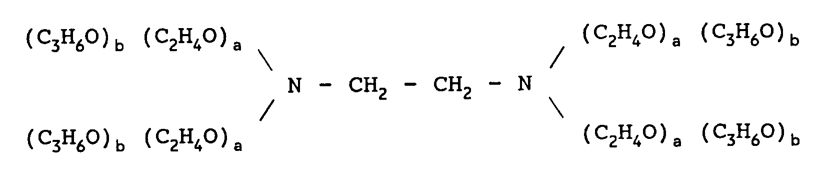 où :
a est un nombre tel que la partie hydrophile représentée par le polyoxyéthylène (C₂H₄O)ₐ (POE) constitue entre 10% et 40% du poids moléculaire total du composé;
le poids moléculaire de l'agrégat moyen de la partie hydrophobe du copolymère octaséquencé consistant en du polyoxypropylène (C₃H₆O)_{b} (POP) se situe entre 4000 et 9000 daltons, et
b est un nombre tel que la partie polyoxypropylène (C₃H₆O)_{b} (POP) du composé constitue entre 60% et 90% du poids moléculaire total du copolymère octaséquencé.

12. Procédé suivant la revendication 10, où l'adjuvant a la formule suivante : 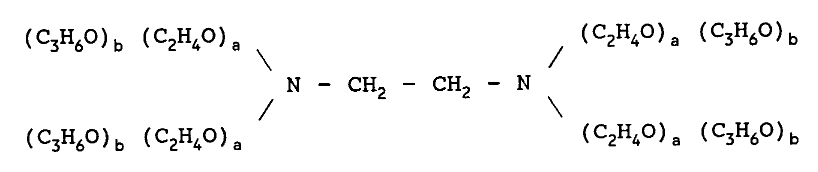 où, a est égal à 5 et b est égal à 32.

13. Procédé suivant la revendication 10, où l'adjuvant a la formule suivante :
HO(C₂H₄O)ₐ (C₃H₆O)_{b} (C₂H₄O)ₐ H
où, le poids moléculaire du (C₃H₆O) hydrophobe se situe entre 2000 et 5000 et le poids moléculaire total du composé se situe entre 2300 et 6000.

14. Procédé suivant la revendication 10, où l'adjuvant a la formule suivante :
HO(C₂H₄O)ₐ (C₃H₆O)_{b} (C₂H₄O)ₐ H
où, le poids moléculaire du (C₃H₆O) hydrophobe est d'environ 4300 et le pourcentage du (C₂H₄O)ₐ hydrophile est d'environ 10% en poids.

15. Utilisation d'une protéine de flagelline bactérienne polymérisée conjuguée à un antigène non salmonella pour la production d'un vaccin contre l'antigène pour l'immunisation d'un humain ou animal, où l'antigène est choisi parmi le groupe consistant en des peptides, des polysaccharides, des glycopeptides, des médicaments et des haptènes de poids moléculaire faible.

16. Utilisation suivant la revendication 15, où le vaccin est combiné à un adjuvant.

17. Utilisation suivant la revendication 16, où l'adjuvant a la formule suivante : 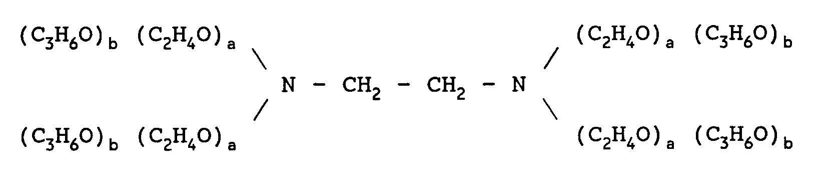 où :
a est un nombre tel que la partie hydrophile représentée par le polyoxyéthylène (C₂H₄O)ₐ (POE) constitue entre 10% et 40% du poids moléculaire total du composé;
le poids moléculaire de l'agrégat moyen de la partie hydrophobe du copolymère octaséquencé consistant en du polyoxypropylène (C₃H₆O)_{b} (POP) se situe entre 4000 et 9000 daltons, et
b est un nombre tel que la partie polyoxypropylène (C₃H₆O)_{b} (POP) du composé constitue entre 60% et 90% du poids moléculaire total du copolymère octaséquencé.

18. Utilisation suivant la revendication 16, où l'adjuvant a la formule suivante : 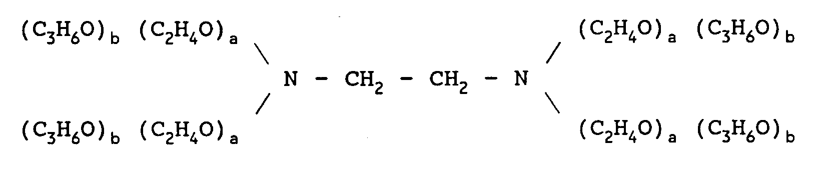 où, a est égal à 5 et b est égal à 32.

19. Utilisation suivant la revendication 16, où l'adjuvant a la formule suivante :
HO(C₂H₄O)ₐ (C₃H₆O)_{b} (C₂H₄O)ₐ H
où, le poids moléculaire du (C₃H₆O) hydrophobe se situe entre 2000 et 5000 et le poids moléculaire total du composé se situe entre 2300 et 6000.

20. Utilisation suivant la revendication 16, où l'adjuvant a la formule suivante :
HO(C₂H₄O)ₐ (C₃H₆O)_{b} (C₂H₄O)ₐ H
où, le poids moléculaire du (C₃H₆O) hydrophobe se situe à environ 4300 et le pourcentage du (C₂H₄O)ₐ hydrophile est d'environ 10% en poids.
